# EUROPEAN PATENT SPECIFICATION

(11) **EP 1 350 459 B1**
(45) Date of publication and mention of the grant of the patent: **21.06.2006**
(21) Application number: 03425213.0
(22) Date of filing: 04.04.2003
(51) Int. Cl.: A61B 1/24

(54) **Control device for an electronic viewer for dental surgery**
Kontrollvorrichtung für einen electronischen Sucher für dentale Chirurgie
Dispositif de contrôle d'un vidéoendoscope pour la chirurgie dentaire

(30) Priority: 05.04.2002 IT BO20020178
(43) Date of publication of application: 08.10.2003
(73) Proprietor: CASTELLINI S.p.A., 40013 Castel Maggiore (Bologna) (IT)
(72) Inventor: Castellini, Franco, 40124 Bologna (IT)
(74) Representative: Lanzoni, Luciano

(56) References cited:
- WO-A-99/27865
- US-A- 5 751 341
- US-A- 5 908 294
- US-B1- 6 217 512

## Description

The present invention relates to a control device for a visual imaging handpiece, in particular for dental surgeries.

At present, the high quality achieved in multimedia equipment, in terms of both reliability and digital viewing specifications and the capacity to save any data needed by the user and operator, has resulted in increasing demand for apparatus dedicated to dental checks. We refer, in particular, to apparatus which can help the dental surgeon perform an immediate check of the patient's mouth, but also catalogue all data relating to the patient using magnetic archives.

The apparatus most widely used for this purpose consists of a camera handpiece designed to record intraoral visual images of the patient's mouth.

This handpiece basically consists (see also patent EP - 282.832 and patent IT - 1.274.909, the latter by the same Applicant) of a known micro-camera enclosed or supported in a relative housing / handling element and connected to a viewing apparatus which may, in turn, be part of an image recording system (a known video and relative hardware unit and support software). A knows example of such device is given by document WO 99/27865.

The operating zone or end in which the micro-camera is fitted also has a plurality of lighting terminals, suitably powered, and in a first solution consisting of fibre optic units, arranged close to the micro-camera lens.

In a more up-to-date solution, these lighting terminals consist of a plurality of LEDs, reducing the overall dimensions of the handpiece.

Therefore, the dental surgeon can use such a handpiece for general "screening" in the patient's mouth and, if necessary, to record the images of any parts of greater interest to the dental surgeon for the treatment carried out at that time or to be carried out.

However, one problem encountered on such camera handpieces is the possibility that the micro-camera image may lack immediate sharpness due to blurring of the micro-camera lens at the moments when imaging starts, since activation of the lighting (in particular with the LEDs), normally done when the handpiece is picked up from its seat, causes a change in temperature, that is to say a ΔT, which is high and concentrated in a limited visual imaging area.

This situation, which may arise if the camera is in an atmosphere with a controlled temperature, or at the start of a session, or with the handpiece at room temperature, results in temporary blurring which inevitably affects the time required for the dental surgeon to make images inside the patient's mouth.

The aim of the present invention is, therefore, to overcome the above-mentioned disadvantages by providing a control device for a visual imaging handpiece, in particular applicable on dental units, which is extremely practical, operates immediately and provides high quality images.

This aim is fulfilled by a control device as claimed in one or more of the enclosed claims.

The technical features of the present invention, in accordance with the above-mentioned aims, are set out in the claims herein and the advantages more clearly illustrated in the detailed description which follows, with reference to the accompanying drawings, which illustrate a preferred embodiment of the invention without limiting the scope of the inventive concept, and in which:
- Figure 1 is a block diagram of a visual imaging handpiece, in particular for making intraoral images of patients' mouths in dental surgeries, fitted with the control device made according to the present invention;
- Figure 2 is an enlarged front view of a detail of the handpiece illustrated in Figure 1;
- Figure 3 is a graph showing operation of the intraoral visual imaging handpiece controlled by the device disclosed.

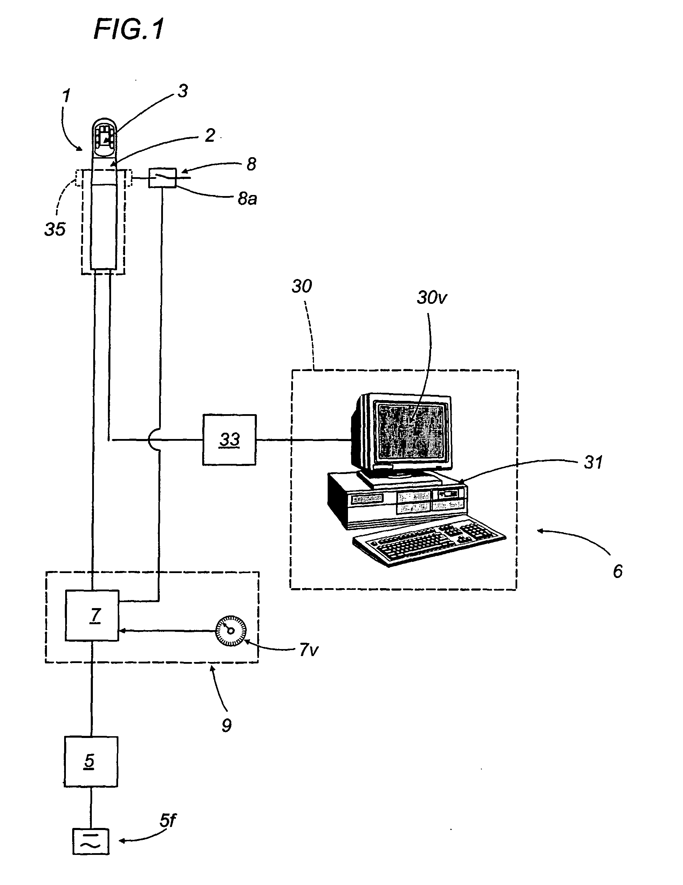

With reference to the accompanying drawings, and in particular with reference to Figure 1, the device disclosed is used to control a visual imaging handpiece, in particular for making intraoral images of patients' mouths in dental surgeries.

This handpiece, labelled 1, comprises a grip body 2 with, at a first end, a visual imaging terminal 3, for example a known CCD, that is to say, Charge Coupled Device with digital interface 33, not illustrated in detail here because it is of the known type. The CCD unit is located in an optical system consisting of a prism, a diaphragm and a lens.

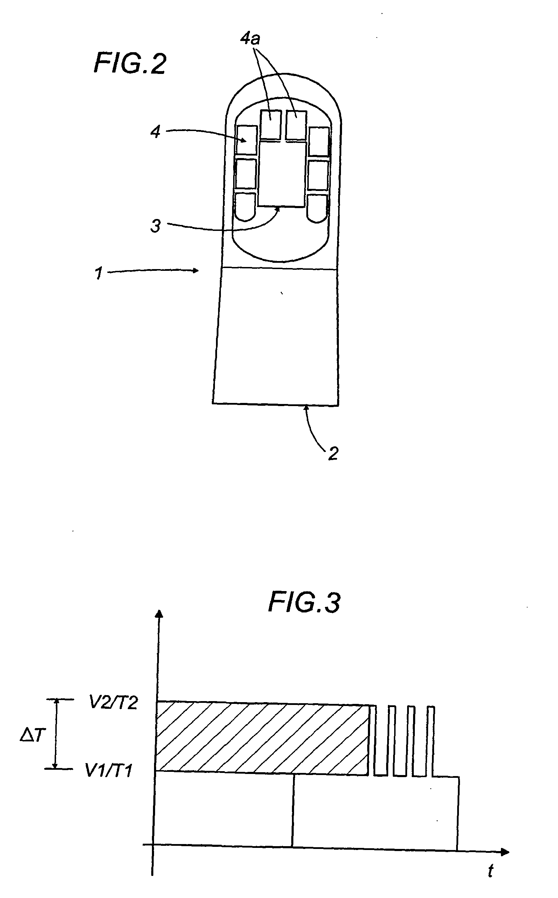

The numeral 4 denotes lighting means (see also Figure 2) at least in the terminal 3 imaging zone and arranged around the optical system.

The terminal 3 is connected, at one end of the grip body 2, to means 6 for viewing and/or recording the images, which may consist of a hardware system 30 comprising a viewer 30v for images made by the terminal, a processor 31 for the images and archives, for example, of the magneto-optical type for the images made.

The above-mentioned lighting means 4 are controlled by power supply means 5 (a known electrical power supply unit) connected to an energy source 5f. The lighting means 4 may consist of a plurality of LEDs 4a (see Figure 2) arranged around the edge of the imaging terminal 3.

Imaging terminal 3 switching on and switching off (and, therefore, also switching on and off the LEDs 4a) are controlled by second control means 8 acting on the terminal 3 and consisting, in a first embodiment illustrated in Figure 1, of a push-button on a support 35 which can house the grip body 2 in such a way that the button can be kept in a position in which it keeps the circuit open and, obviously, is activated when the grip body 2 is picked up to record images.

In another embodiment, illustrated in Figure 2, there may be a switch 8a on the grip body 2. Like the previous one, the switch 8a may be kept in a position in which it keeps the circuit open, for example, by resting the handpiece 1 on the support 35 which may be part of the equipment supplied, or it may be present on a dental unit in the surgery (not illustrated here), and the circuit can be activated when the grip body 2 is picked up from the support 35.

The numeral 7 denotes first control means acting on the power supply means 5, and designed to allow predetermined adjustment of the brightness of the lighting means 4, that is to say, of the temperature value which can be reached by the lighting means 4.

These first control means 7 comprise an adjusting unit 7, inserted between the second control means 8 and the power supply means 5 so as to allow the lighting means 4 to achieve a maximum brightness (V₂ / T₂), when the terminal 3 is switched on (that is to say, when the grip body 2 is removed from the support 35), and to allow the lighting means 4 to achieve a minimum brightness (V₁ / T₁), when the terminal 3 is switched off (when the grip body 2 is returned to the support 35) and vice versa.

If, as indicated, the dental surgery has a dental unit, which may be fitted with a microprocessor unit 9 (illustrated with a dashed line in Figure 1) which controls and activates primary and auxiliary functions of the apparatus present on the dental unit, the imaging terminal 3, the lighting means 4, the power supply means 5 and first control means 7 for the power supply means 5 can be directly controlled by the microprocessor unit 9.

More precisely, the microprocessor unit 9 may form the first control means 7 for the power supply means 5 and the microprocessor unit 9 is connected to the second means 8 for switching on and off the terminal 3 and the lighting means 4 located on the grip body 2.

This allows adjustment, that is to say, variations in, the brightness of the lighting means 4 from the above-mentioned minimum brightness to the maximum brightness, and vice versa directly from the dental unit.

In practice, as is also apparent in Figure 3, the first control means 7, comprising for example, as the adjusting unit 7v a potentiometer for adjusting maximum and minimum brightness values, can provide lighting continuity, within a given range, so as to avoid completely switching off the LEDs 4a.

This continuity may be seen in the graph, in which the y-axis shows a power value V and equivalent temperature T which can be reached by the LEDs 4a and the x-axis shows a time t reaching to infinity.

Observation of the power / temperature curve reveals that during the periods when the handpiece 1 is not used, the value V₁ / T₁ is greater than zero and is constant, whilst during the periods in which the handpiece is used 1 a value V₂ / T₂ greater than the previous one is rapidly reached, which is present until the handpiece 1 is switched off.

The "range" or temperature difference indicated with ΔT in Figure 3 is between V₁ / T₁ and V₂ / T₂, which may vary according to the ambient conditions in the dental surgery, meaning that the lens of the terminal 3 for making intraoral images of the patients' mouths can be kept as sharp as possible during the transition between the non-operating position and the operating or imaging position, so that high-quality intraoral images can be made immediately.

Therefore, the device disclosed fulfils the preset aims thanks to a simple and rational handpiece structure, in which the apparatus is not weighed down and with simple adjustment of the power supply to the lighting means, so that the camera is always in the optimum condition for making images.

## Claims

1. In combination, a visual imaging handpiece (1) and a control device for a said visual imaging handpiece, in particular for making intraoral images of patients' mouths in dental surgeries; the handpiece (1) comprising a grip body (2) with, at a first end, a visual imaging terminal (3) and lighting means (4), controlled by power supply means (5), at least for the visual imaging terminal (3) and, at the other end, the grip body (2) being connected to means (6) for viewing and/or recording the images made by the terminal (3), the control device comprising first control means (7) acting on the power supply means (5) and designed to allow predetermined adjustment of the brightness of the lighting means (4), that is to say, of the temperature value which can be reached by the lighting means (4),
in which the imaging terminal (3) is controlled by second control means (8) acting on the terminal (3) and designed to allow the terminal to be switched on or off, the device being **characterised in that** the first control
means (7) comprise an adjusting unit (7v) inserted between the second control means (8) and the power supply means (5), allowing the lighting means (4) to reach maximum brightness when the terminal (3) is switched on, and allowing the lighting means (4) to reach minimum brightness when the terminal (3) is switched off.

2. The device according to claim 1, in which the imaging terminal (3), the lighting means (4), the power supply means (5) and the first control means (7) are controlled by a microprocessor unit (9) of a dental unit.

3. The device according to claim 2, **characterised in that** the microprocessor unit (9) constitutes the first control means (7) and is connected to a switch (8a) located on the grip body (2) for switching on /off the terminal (3) and the lighting means (4), so as to adjust, that is to say vary, the brightness of the lighting means (4) from a minimum brightness to a maximum brightness and vice versa.

4. The device according to claim 2, **characterised in that** the microprocessor unit (9) constitutes the first control means (7) and is connected to a button located on a supporting element (35) which houses the grip body (2) for switching on / off the terminal (3) and the lighting means (4), thus adjusting, that is to say varying, the brightness of the lighting means (4) from a minimum brightness to a maximum brightness and vice versa.

5. The device according to claim 3 or 4, **characterised in that** the first control means (7) for the power supply means (5) provide lighting continuity, within a given range, so as to avoid completely switching off the lighting means (4).

6. The device according to claim 1, **characterised in that** the adjusting unit (7v) comprises a potentiometer acting on the power supply means (5).

## Patentansprüche

1. Kombination eines Video-Handstückes (1) und einer Steuervorrichtung für ein genanntes Video-Handstück, insbesondere zum Aufnehmen von intraoralen Bildern im Mund des Patienten bei zahnärztlichen Eingriffen; wobei das Handstück (1) einen Griffkörper (2) enthält, welcher an einem freien Ende ein Videoterminal (3) und Beleuchtungsmittel (4) trägt, gesteuert durch Speisungsmittel (5), wenigstens für das Videoterminal (3), und wobei an dem anderen Ende der Griffkörper (2) an Mittel (6) zum Sichtbarmachen und/oder zum Aufzeichnen der durch das Terminal (3) aufgenommenen Bilder angeschlossen ist, wobei die Steuervorrichtung erste Steuermittel (7) enthält, die auf die Speisungsmittel (5) wirken und dazu bestimmt sind, eine vorgegebene Regulierung der Helligkeit der Beleuchtungsmittel (4) zu erlauben, das heisst des Temperaturwertes, welcher durch die Beleuchtungsmittel (4) erreicht werden kann, bei welcher das Videoterminal (3) durch zweite Steuermittel (8) gesteuert wird, die auf das Terminal (3) wirken und dazu bestimmt sind, das Ein- oder Ausschalten des Terminals zu erlauben, wobei die Vorrichtung **dadurch gekennzeichnet ist, dass** die ersten Steuermittel (7) eine Reguliereinheit (7v) enthalten, eingesetzt zwischen den zweiten Steuermitteln (8) und den Speisungsmitteln (5), wodurch es den Beleuchtungsmitteln (4) ermöglicht ist, die maximale Helligkeit zu erreichen, wenn das Terminal (3) eingeschaltet ist, und es den Beleuchtungsmitteln (4) ermöglicht ist, die minimale Helligkeit zu erreichen, wenn das Terminal (3) ausgeschaltet ist.

2. Vorrichtung nach Patentanspruch 1, bei welcher das Video-Terminal (3), die Beleuchtungsmittel (4), die Speisungsmittel (5) und die ersten Steuermittel (7) durch eine Mikroprozessoreinheit (9) der zahnärztlichen Einheit gesteuert werden.

3. Vorrichtung nach Patentanspruch 2, **dadurch gekennzeichnet, dass** die Mikroprozessoreinheit (9) die ersten Steuermittel (7) bildet und an einen an dem Griffkörper (2) angeordneten Schalter (8a) zum Ein- und Ausschalten des Terminals (3) und der Beleuchtungsmittel (4) angeschlossen ist, so dass die Helligkeit der Beleuchtungsmittel (4) von einer minimalen Helligkeit auf eine maximale Helligkeit und umgekehrt reguliert, beziehungsweise verändert werden können.

4. Vorrichtung nach Patentanspruch 2, **dadurch gekennzeichnet, dass** die Mikroprozessoreinheit (9) die ersten Steuermittel (7) bildet und an einen Knopf angeschlossen ist, angeordnet an einem Halteelement (35), welches den Griffkörper (2) aufnimmt, um das Terminal (3) und die Beleuchtungsmittel (4) ein- und auszuschalten, so dass die Helligkeit der Beleuchtungsmittel (4) von einer minimalen Helligkeit auf eine maximale Helligkeit und umgekehrt reguliert, beziehungsweise verändert werden können.

5. Vorrichtung nach Patentanspruch 3 oder 4, **dadurch gekennzeichnet, dass** die ersten Steuermittel (7) für die Speisungsmittel (5) eine Kontinuität der Beleuchtung innerhalb eines gewissen Bereiches vorsehen, so dass ein vollständiges Ausschalten der Beleuchtungsmittel (4) vermieden wird.

6. Vorrichtung nach Patentanspruch 1, **dadurch gekennzeichnet, dass** die Reguliereinheit (7v) ein Potentiometer enthält, das auf die Speisungsmittel (5) wirkt.

## Revendications

1. En combinaison, une pièce à main d'imagerie vidéo (1) et un dispositif de contrôle de ladite pièce à main d'imagerie vidéo, en particulier pour la prise d'images intraorales de la bouche du patient pour la chirurgie dentaire ; la pièce à main (1) comprenant un corps de préhension (2) avec, à une première extrémité, un terminal d'imagerie vidéo (3) et des moyens d'éclairage (4) commandés par des moyens d'alimentation électrique (5), au moins pour le terminal d'imagerie vidéo (3), le corps de préhension (2) étant relié, à l'autre extrémité, à des moyens (6) de visualisation et/ou d'enregistrement des images filmées par le terminal (3), le dispositif de contrôle comprenant des premiers moyens de commande (7) agissant sur les moyens d'alimentation électrique (5) et destinés à permettre un réglage prédéfini de la luminosité des moyens d'éclairage (4), c'est-à-dire, de la valeur de température qui peut être atteinte par les moyens d'éclairage (4), dans lequel le terminal d'imagerie (3) est commandé par des deuxièmes moyens de commande (8) agissant sur le terminal (3) et destinés à permettre la mise en marche ou l'arrêt du terminal, le dispositif étant **caractérisé en ce que** les premiers moyens de commande (7) comprennent une unité de réglage (7v) interposée entre les deuxièmes moyens de commande (8) et les moyens d'alimentation électrique (5), permettant aux moyens d'éclairage (4) d'atteindre une luminosité maximale quand le terminal (3) est mis en marche, et permettant aux moyens d'éclairage (4) d'atteindre une luminosité minimale quand le terminal (3) est éteint.

2. Le dispositif selon la revendication 1, dans lequel le terminal d'imagerie (3), les moyens d'éclairage (4), les moyens d'alimentation électrique (5) et les premiers moyens de commande (7) sont contrôlés par une unité à microprocesseur (9) d'une unité dentaire.

3. Le dispositif selon la revendication 2, **caractérisé en ce que** l'unité à microprocesseur (9) constitue les premiers moyens de commande (7) et est connectée à un interrupteur (8a) situé sur le corps de préhension (2) pour mettre en marche ou arrêter le terminal (3) et les moyens d'éclairage (4), de façon à régler, c'est-à-dire à faire varier, la luminosité des moyens d'éclairage (4) d'une luminosité minimale à une luminosité maximale et inversement.

4. Le dispositif selon la revendication 2, **caractérisé en ce que** l'unité à microprocesseur (9) constitue les premiers moyens de commande (7) et est connectée à un bouton situé sur un élément de support (35) qui reçoit le corps de préhension (2) pour la mise en marche ou l'arrêt du terminal (3) et des moyens d'éclairage (4), réglant ainsi, c'est-à-dire faisant ainsi varier la luminosité des moyens d'éclairage (4) d'une luminosité minimale à une luminosité maximale et inversement.

5. Le dispositif selon la revendication 3 ou 4, **caractérisé en ce que** les premiers moyens de commande (7) des moyens d'alimentation électrique (5) fournissent un éclairage continu, dans une plage donnée, afin d'éviter l'arrêt total des moyens d'éclairage (4).

6. Le dispositif selon la revendication 1, **caractérisé en ce que** l'unité de réglage (7v) comprend un potentiomètre agissant sur les moyens d'alimentation électrique (5).
